# EUROPEAN PATENT APPLICATION

(11) **EP 3 738 588 A1**
(43) Date of publication of application: **18.11.2020**
(21) Application number: 20174271.5
(22) Date of filing: 12.05.2020
(51) Int. Cl.: A61K 31/185, A61K 33/06, A61K 33/08, A61K 9/00, A61K 8/00, A61K 9/06, A61P 17/00, A61K 45/06

(54) **TOPICAL AND ORAL FORMULATIONS COMPRISING TAURINE AND MAGNESIUM FOR USE IN THE TREATMENT OF ROSACEA**

(30) Priority: 13.05.2019 US 201916411025
(71) Applicant: Profet, Margaret Jean, Manhattan Beach, CA 90266 (US)
(72) Inventor: PROFET, Margaret Jean, Manhattan Beach, CA 90266 (US)
(74) Representative: J A Kemp LLP

(57) **Abstract**

Combinations and methods are disclosed for treating and/or preventing rosacea, a disorder of the sebaceous glands/ducts of the pilosebaceous units and a disorder of the stratum corneum. The combinations comprise effective amounts of taurine and effective amounts of magnesium, wherein the combinations of the effective amounts of taurine and magnesium are sufficient to help normalize sebaceous gland/duct function and stratum corneum function, and thereby treat and/or prevent the rosacea.

## Description

### PRIORITY AND CROSS REFERENCE TO RELATED APPLICATIONS

This application claims priority to U.S. Application No. 16/411,025, filed May 13, 2019, the entire disclosure of which is incorporated herein by reference in its entirety. This application is a Continuation-In-Part Application of EP Application No. 16812186, which claims priority to U.S. Application No. 15/179,815, filed Jun. 10, 2016, now U.S. Pat. No. 9,795,633, issued on Oct. 24, 2017, which claims priority to U.S. Provisional Application No. 62/180,842, filed Jun. 17, 2015, the entire disclosures of which are incorporated herein by reference thereto in their entireties.

### BACKGROUND

Rosacea is a facial skin disorder of the pilosebaceous units and the stratum corneum. It is characterized at the mild level mainly by facial redness, thickening of the skin of certain facial regions, acne papules and pustules, inflammation, and purplish broken or otherwise visible blood vessels near the surface of the skin. At the more severe level it is often characterized in addition by severe inflammation, a greatly enlarged nose, or redness of the eyes and poor eye lubrication. Rosacea afflicts millions of people in the world each year, most commonly adults older than 30 years.
The 4 main subvariants of rosacea are widely considered to be:
Erythematotelangiectatic - facial erythema especially in the nasal region, with some visible broken blood vessels;
Papulopustular - facial redness with patches of skin thickening and acne papules and/or pustules;
Sebaceous hyperplasia of the nasal region, sometimes with marked rhinophyma - red granulomatous bulbous nose;
Ocular - obstruction of the Meibomian glands of the eyes, leading to poor tear film layer and to ocular dryness and redness and sometimes to vision impairment.

The etiology of rosacea is multi-factorial, and rosacea is still considered in the medical field to be an unsolved problem overall, lacking definitive cures. The development of a new treatment that helps resolve or alleviate some of the symptoms of rosacea, including 'acne rosacea', or that prevents rosacea from worsening to another subvariant of rosacea would be of significant benefit to patients.
Rosacea has some symptoms and some etiological components in common with acne, which is why treatments for acne are sometimes prescribed for rosacea. But most such treatments are not highly effective for rosacea, in part because they are usually not highly effective for acne either, and in part because rosacea is complex in ways different from acne. There is a great need for an effective rosacea treatment that can simultaneously help normalize the affected facial sebaceous glands and sebaceous ducts of the pilosebaceous units and also help normalize the affected facial stratum corneum.

### SUMMARY

A combination is disclosed for treating and/or preventing rosacea. The combination includes an effective amount of taurine and an effective amount of magnesium, wherein the combination of the effective amounts of taurine and magnesium is sufficient to partially or fully normalize sebaceous gland and sebaceous duct function and stratum corneum function and to thereby treat and/or prevent the rosacea.

In some embodiments, the effective amounts of taurine and magnesium are in a dosage form selected from an oral or topical dosage form. The oral dosage form may be selected from a capsule, a tablet, or a unit dose of powder. The topical dosage form may be selected from a gel or a cream. In one embodiment of the oral dosage form, the effective amount of taurine is in a range of 500 mg to 6000 mg per unit dose, and the effective amount of magnesium is in a range of 25 mg to 500 mg per unit dose. In one embodiment of the topical dosage form, the effective amount of taurine is in a range of 200 mg to 2 g per ½ teaspoon, and the effective amount of magnesium is in a range of 40 mg to 400 mg per ½ teaspoon.

In some embodiments, the effective amounts of taurine and magnesium are provided together in a single oral dosage form. In some embodiments, the effective amounts of taurine and magnesium are provided together in a single topical dosage form.

In one embodiment, the combination further comprises a pharmaceutically acceptable carrier.

In one embodiment, the combination further comprises one or more micronutrients selected from the group consisting of vitamin D, vitamin A, zinc, choline, vitamin B1, vitamin B2, vitamin B3, vitamin B5, vitamin C, vitamin E, vitamin K, folic acid, calcium, iron, phosphorous, iodine, potassium, selenium, manganese, copper, inositol, omega 3 fatty acids, lycopene, lutein, and zeaxanthin.

A method is disclosed in accordance with certain embodiments for treating and/or preventing rosacea in a subject in need thereof. The method comprises administering to the subject an effective amount of taurine and an effective amount of magnesium, simultaneously or sequentially, wherein the effective amounts are sufficient in combination to treat and/or prevent the skin condition.

In some embodiments, the effective amounts of taurine and magnesium are provided in an oral dosage form selected from a capsule, a tablet, or a unit dose of powder, formulated for oral administration. In certain embodiments of the oral dosage form, the effective amount of taurine is in a range of 500 mg to 6000 mg per unit dose and the effective amount of magnesium is in a range of 25 mg to 500 mg per unit dose.

In some embodiments, the effective amounts of taurine and magnesium are provided in a topical dosage form selected from a gel or a cream, formulated for topical administration. In certain embodiments of the topical dosage form, the effective amount of taurine is in a range of 200 mg to 2 g per ½ teaspoon, and the effective amount of magnesium is in a range of 40 mg to 400 mg per ½ teaspoon.

In some embodiments, the oral dosage form is configured for a single daily dose or multiple daily doses.

In some embodiments, the topical dosage form is configured for a single daily dose or multiple daily doses.

In some embodiments, the method for treating and/or preventing rosacea further comprises administering one or more micronutrients selected from the group consisting of vitamin D, vitamin A, zinc, choline, vitamin B1, vitamin B2, vitamin B3, vitamin B5, vitamin C, vitamin E, vitamin K, folic acid, calcium, iron, phosphorous, iodine, potassium, selenium, manganese, copper, inositol, omega 3 fatty acids, lycopene, lutein, and zeaxanthin.

### DETAILED DESCRIPTION

Taurine is a free amino acid synthesized by the body as well as obtained in diet through meat, fish, and milk. It is not one of the alpha amino acids that serve as building blocks for proteins. Most commercially available taurine is synthesized in laboratories. Magnesium is a mineral. Taurine and magnesium each have many different functions in the body depending on such factors as cell type and target protein, and correspondingly, taurine and magnesium have many different mechanisms of action to perform those various functions.

Taurine and magnesium have been found to work synergistically, in that taurine can facilitate the transport of magnesium across cell membranes.

The combination of taurine and magnesium (referred to herein generally as "TAUMAG", and also referred to herein specifically as "TAUMAG-R", where "R" refers to "rosacea"), is likely to be effective against features of rosacea that are in common with acne, because TAUMAG is effective against acne, and the TAUMAG mechanisms of action that work against acne should similarly work against rosacea.

Surprisingly, TAUMAG is also likely to be effective against some of the features of rosacea that are not in common with acne, because some of the additional mechanisms of action of TAUMAG coincidentally happen to work against some of the other major symptoms of rosacea. Some subvariants of rosacea are described in the Examples.

The following list shows the main features of rosacea that are in common with acne versus the main features of rosacea not in common with acne:

Acne is a disorder of the pilosebaceous units, whereas rosacea is a disorder involving the pilosebaceous units and also the stratum corneum barrier of the skin.

Acne primarily involves the abnormal production and flow of sebaceous lipids, whereas rosacea can involve abnormal production and flow both of the sebaceous lipids and of the epidermal lipids of the stratum corneum.

Both acne and rosacea often involve, secondarily, infiltration of skin structures by microorganisms that feed on the skin lipids, but the main species of micro-organism exacerbating acne, C. acnes (P. acnes), are different from the main species exacerbating rosacea, Demodex spp. and H. pylori.

Both acne and rosacea often involve inflammation, but the inflammation is generally more pronounced in rosacea.

### Rosacea Treatment with TAUMAG-R

Whereas the goal with TAUMAG treatment for acne is to restore to normal range the sebum production and flow, the goal with TAUMAG-R treatment for rosacea is to restore to normal range the sebum production and flow, the epidermal lipid production and flow, and the stratum corneum barrier function (taking into account that what is a normal lipid composition of someone of typical acne age, young, is not necessarily what is a normal lipid composition of someone of typical rosacea age, middle-aged to old.)

With TAUMAG-R treatment for rosacea, the array of different TAUMAG-R mechanisms of action can help alleviate 2 categories of rosacea problems:

### 1. TAUMAG-R for Rosacea Problems of the Pilosebaceous Units

As shown in parent U.S. Pat. No. 9,795,633, its U.S. Continuation Patent 10,342,823, and parent EP Pat. Application No. 16812186, taurine inhibits lipogenesis within the sebaceous glands, thereby decreasing the volume of sebum in the sebaceous glands, sebaceous ducts, or both; and the magnesium breaks down the large lipid droplets of the sebaceous glands, sebaceous ducts, or both, thereby enabling sebum to flow with less chance of clogging the sebaceous ducts.

Taurine in the sebaceous glands likely helps stop excess lipogenesis, by binding as an inhibitory ligand to the LXR-alpha (liver X receptor-alpha). The LXR-alpha is a receptor that is expressed by many cell types, including cells of the sebaceous glands, and that usually induces lipogenesis when activated by a ligand. The main evidence that taurine halts LXR-alpha-induced lipogenesis is a study by Hoang et al., Molecular Nutrition & Food Research, 56:900-911 (2012) showing experimentally in liver cells in vitro that taurine is a ligand of LXR-alpha that inhibits lipogenesis, resulting in a dose-dependent marked reduction in cellular lipid levels. Taurine is likely to have the same LXR-alpha-related function of reducing lipogenesis in sebaceous glands that it has in liver cells. A reduction of lipogenesis in the sebaceous glands results in a reduction of sebum and consequently less chance of sebum clogging the pilosebaceous units. Taurine thus appears to be part of the regulatory feedback system that keeps sebaceous lipogenesis in check.

Magnesium likely helps activate the breakdown of the large lipid droplets in the sebaceous glands/sebaceous ducts via magnesium-activated protein kinase phosphorylation of the lipid droplet surface protein perilipin. This process allows perilipin to direct lipolysis of lipid droplets, fragmenting them into many microlipid droplets and dispersing them. Support for this activity of magnesium comes from the following two pieces of information taken together: protein kinase A phosphorylation of perilipin A has been shown in fat cells to drive the rapid fragmentation and dispersion of lipid droplets (A. Marcinkiewicz et al., Journal of Biological Chemistry, 281:11901-11909 (2006)); magnesium plays an activating role in protein kinase A phosphorylation (Adams, Chemical Reviews, 101:2271-2290 (2001)). Magnesium likely has the same effect of activating lipolysis of the large perilipin-coated lipid droplets in the sebaceous glands/ducts that it does of the lipid droplets of fat cells. A fragmentation of the large lipid droplets into micro-droplets may help prevent the clogging of the pilosebaceous units. Magnesium may also help trigger the breakdown of lipid droplets in that PKA phosphorylation of perilipin A results in the activation of lipases and their recruitment to the surfaces of lipid droplets (Bickel, 2009; Sahu-Osen et al., 2015; Sztalryd et al, 2003). In addition, magnesium also appears to contribute to sebum synthesis inhibition by intervening via protein inactivation at specific points along the biochemical pathways of sebum synthesis, possibly also altering the sebum lipid composition. In the pathways of sebum synthesis, for fatty acid/triacylglycerol synthesis the rate-limiting step is the enzyme ACC (acetyl coA carboxylase), and for squalene/cholesterol synthesis the rate-limiting step is the enzyme HMG-coA-reductase. Magnesium action on ACC inhibits it (Hardie and Pan, 2002), and on HMG-coA-reductase inhibits it (Friesen and Rodwell, 2004; Rosanoff and Seelig, 2004). Magnesium intervention can also lead to a decrease in sebaceous gland size: magnesium catalyzes AMPK protein kinase phosphorylation, and whereas the sebaceous lipid droplet protein PLIN2 stimulates sebaceous gland growth and proliferation of sebocytes (Dahlhoff et al, 2013), AMPK protein kinase phosphorylation of PLIN2 triggers its degradation (Kaushik and Cuervo, 2016).

Such mechanisms of action in the pilosebaceous units indicate that TAUMAG-R might be especially therapeutic in reversing papulopustular rosacea and sebaceous gland hyperplasia of rosacea. In particular, there is a need for a rosacea treatment that directly inhibits excess sebum synthesis and sebocyte proliferation, and that prevents clogging of the sebaceous ducts without injuring or otherwise undermining basic sebaceous gland function. Currently the only treatments that significantly inhibit sebum synthesis also inhibit sebaceous gland function, sometimes with permanent injury to the sebaceous glands (e.g., isotretinoin or laser ablation). Such treatments tend to change the sebaceous glands from one abnormal state to another abnormal state rather than achieving an optimal state of sebaceous activity in the range of normal.

### 2. TAUMAG-R for Rosacea Problems of the Stratum Corneum Barrier

Rosacea concerns the convexities of the central face-i.e., where the outer epithelial cells bend, particularly the nose, and also the cheeks, chin, and forehead (Vermuri et al, 2015).

Epithelial cells of outer surfaces, especially the stratum corneum, provide barrier protection via cell arrangements with tight junctions between the cells and with molecular gates for selective permeability (Zihni et al, 2016). But these tight cellular arrangements must also be able to flexibly bend in anisotropic ways to follow the contours of the various organs they surround, without leaving dysfunctional gaps between the cells, and while minimizing energy.

This flexibility-curvature-tightness problem in 3-dimensional cell packing arrangements has been so important in biological evolution that nature solved it with special multi-faceted flexible geometrical cell shapes: "scutoids", the recently-discovered polygonal shapes for epithelial cells whose bounding surfaces are anisotropically curved (Gomez-Galvez et al, 2018). The scutoid shapes have been found experimentally in curved epithelial tissues of certain nonmammalian animals, and they are strongly predicted to exist in human epithelial cell arrangements at bending contours, since mathematical modeling and physics simulations show the scutoids to be the most energetically favorable geometric cell shapes for cell arrangements over bended contours (Mughal et al, 2018).

However, even if the convex areas of the human face have these optimal scutoid cell arrangements for a protective barrier, in order for such arrangements to function properly the individual cell volume regulatory functions and paracellular signaling functions need to work well. In rosacea patients, for reasons not fully known, the cell packing arrangements of the curved areas of the face appear to be disrupted--i.e., the stratum corneum barrier protection functions are not working properly.

Some of the TAUMAG-R mechanisms of action are likely to restore the cell packing attributes of the facial epidermis toward normal and to thereby reduce the severity of rosacea, for example by:

Maintaining adequate epidermal lipid status: Epidermal lipids of keratinocyte origin fill the spaces between stratum corneum cells, like "cement" for the barrier structure, but allowing selective permeability (Pappas, 2009). Sebaceous gland activity, density, and lipid composition affect the stratum corneum epidermal lipid volume and composition (Tascini et al, 2019). Increased secretion of sebaceous free fatty acids is correlated with decreased secretion of epidermal free fatty acids, affecting the permeability barrier (Ludovici et al, 2018). Decreasing the production of sebaceous sebum with TAUMAG-R may help to normalize the blend of lipids of the skin surface that help make the tight junction arrangements function properly.

Maintaining epidermal tight junctions: Taurine as a major osmolyte is important in helping to preserve tight junction functions in the epidermis, including maintaining proper cell volume by proper cellular water volume and also possibly maintaining proper conformation of tight junction proteins (El-Chami et al, 2018). This means that taurine likely facilitates epidermal cell packing, possibly enabling each individual cell to alter its volume as needed for best fit with its neighboring cells in packing arrangements.

Hydrating skin: Both taurine and magnesium hydrate the skin, overcoming dryness. Taurine, as an osmolyte regulating cell water volume, has a major role in hydrating skin cells (Janeke et al, 2003). Hydration of stratum corneum is important for alleviating certain symptoms of rosacea, as rosacea is often linked to poor skin hydration and transepidermal water loss. Magnesium chloride topically experimentally applied to skin samples has been shown to double skin hydration within six hours (Chandrasekaran, 2016).

Reducing inflammation: Taurine shows some anti-inflammatory effects, in experiments in retinal pigment epithelium in which taurine down-regulated the pathway of PPAR-gamma-dependent pro-inflammatory mediators Inflammation is a significant part of the problem of rosacea. Since PPAR-gamma is expressed in sebaceous and other skin cells, taurine might be therapeutic in suppressing some of the inflammation of rosacea skin. Stimuli for inflammation in rosacea include microbial infestation and transepidermal water loss (Addor, 2016). Magnesium has significant anti-inflammatory effects on skin, via several different biochemical pathways (Chandrasekaran et al, 2014).

"Topical TAUMAG-R" may be as or more effective than "oral TAUMAG-R" for rosacea. Taurine in a topical solution with penetration/permeation enhancers, applied to human skin samples, has been shown to penetrate all layers of the epidermis and dermis (Da Silva et al, 2008). In one study, taurine itself was shown to increase penetration through the stratum corneum, by increasing the osmotic pressure within corneocytes-thereby drawing large amounts of water into the corneocytes (Mueller et al., 2016). Magnesium chloride topically applied to human skin samples has been shown to permeate skin via the hair follicles (shunt diffusion) and through the stratum corneum (bulk diffusion) (Chandrasekaran, 2016; Chandrasekaran et al, 2016), with magnesium permeability increasing rapidly with higher concentration.

Embodiments of this disclosure relate generally to the use of taurine and magnesium in combination for the treatment and/or prevention of rosacea. The combination of taurine and magnesium has not been used before for rosacea. Taurine and magnesium have not been identified before as triggering a sequence of lipid-controlling mechanisms in the pilosebaceous units and/or stratum corneum that may treat and/or prevent rosacea. Taurine and magnesium also have not been identified before as helping hydrate the skin in ways that may prevent rosacea. The combination of taurine and magnesium for acne has been documented in parent EP Pat. Application No. 16812186 and parent US Pat. No. 9,795,633 and its U.S. Continuation Pat No. 10,342,823.

### Uses of Taurine and Magnesium in Non-Skin Medical Conditions

U.S. Pat. No. 5,876,757 A (McCarty) teaches use of magnesium taurate for the treatment of stroke. U.S. Pat. No. 5,776,504 A (McCarty) teaches use of magnesium taurate for prevention and treatment of pre-eclampsia and for acute cardiac conditions. U.S. Pat. No. 4,199,601 A (Durlach) discloses a taurine derivative and divalent metal salts thereof, including magnesium salts for neuromuscular activity reinforcement; neither taurine nor magnesium are disclosed as individual ingredients. The product "Magnesium+Taurine" by Advanced Orthomolecular Research (AOR) (accessible on the World Wide Web at aor.ca) is an oral supplement comprising magnesium and taurine for cardiovascular and neurological support. The product is not directed to the treatment of rosacea or another skin condition. The oral supplement product "Mag-K-Taurine" by AOR provides electrolytes magnesium, potassium, and taurine for the purpose of improving nerve and heart function; it is not a treatment for rosacea or another skin condition, and it does not come in a topical gel/cream form. None of the prior art is based on the unobvious mechanisms of actions of taurine and magnesium in the pilosebaceous units and stratum corneum that are presented in the following section.

### Topical Compositions Comprising Taurine and Magnesium for Rosacea

In some embodiments, a composition for the prevention and/or treatment of rosacea is provided. The composition may be a topical gel or a topical cream or an oral supplement. In some embodiments, the composition comprises at least two active ingredients. The active ingredients may be micronutrients. In some embodiments of the composition, the two active ingredients are taurine and magnesium. In some embodiments, one of the two active ingredients is present at a relatively high concentration. In some embodiments, one of the two active ingredients is present at a relatively low concentration. One of the two active ingredients is produced synthetically in the standard ways.

In some embodiments, the subject is in need of a composition to prevent rosacea. In a variation, a topical composition is administered to the subject. The topical composition administered to the subject may be a topical gel. The topical composition administered to the subject may be a topical cream. In some embodiments, the subject is also administered an oral composition. In other embodiments, the subject is in need of one or both of the topical compositions. In another variation, the subject is in need of one or both of the topical compositions and the oral composition. Alternatively, prevention and/or treatment of the rosacea may be effected by administration of just the oral dosage form.

In some embodiments, the subject is in need of a treatment for rosacea. The subject is in need of a composition for the treatment for rosacea. In some embodiments, a topical composition is administered to the subject. The topical composition administered to the subject may be a topical gel. The topical composition administered to the subject may be a topical cream. In some embodiments, the subject is also administered an oral composition. In other embodiments, the subject is in need of one or both of the topical compositions. In another variation, the subject is in need of one or both of the topical compositions and the oral composition.

Taurine and magnesium are likely involved in mechanisms that reduce lipogenesis in the sebaceous glands of the skin, and activate the breakdown of large lipid droplets of the sebaceous glands or sebaceous ducts. Taurine and magnesium are also likely involved in mechanisms that help normalize and maintain the barrier and hydration functions of the stratum corneum. A topical composition can achieve rapid penetration of taurine and magnesium into skin. Thus, in some embodiments a topical composition is provided. The topical composition comprises taurine and magnesium. In some embodiments, the topical composition comprising taurine and magnesium can achieve rapid penetration of taurine and magnesium into skin. The topical composition may comprise a combination referred to herein as topical TAUMAG-R.

In some embodiments, a topical composition for the prevention of rosacea is provided. In some embodiments, a topical composition for the treatment of rosacea is provided. In some embodiments, the composition is a topical gel or a topical cream that comprises at least two active ingredients. The two active ingredients may be two micronutrients. In some embodiments, the two micronutrients are taurine and magnesium. In some embodiments, taurine is present in high concentrations and magnesium is present in low concentrations. The taurine may be produced synthetically in the standard ways.

In some embodiments, the dose of taurine is about 3 g per day. The dose of taurine may be from about 0.5 to about 6 g per day. In some embodiments, the dose of taurine is equal to about 0.5, 1, 1.5, 2, 2.5, 3, 3.5, 4, 4.5, 5, 5.5 or 6 g per day or within a range defined by any two of the aforementioned values. The dose of taurine in the topical composition may be greater than about 0.1 g per day and less than about 20 g per day. For example, a topical dose of taurine may be from about 0.1 g to about 20 g per day, e.g. from about 0.5 g to about 6 g per day, such as from about 1 g to about 5.5 g per day, e.g. from about 1.5 g to about 5 g per day, such as from about 2 g to about 4.5 g per day, e.g. from about 2.5 g to about 4 g per day, such as from about 3 g to about 3.5 g per day.

In another variation, the topical composition comprises taurine in a range of about 200 mg to about 2 g per 1/2 teaspoon of a topical gel or a topical cream. The topical composition may comprise from about 200 mg to about 2 g taurine per 2.5 mL, such as from about 0.5 g to about 1.5 g per 2.5 mL, e.g. about 1 g taurine per 2.5 mL. The topical composition is preferably a topical gel or topical cream.

The topical composition may additionally comprise a low dose of magnesium. The magnesium in TAUMAG-R may be magnesium citrate, magnesium chloride, magnesium oxide or some other form of magnesium. In some embodiments, the dose of magnesium is about 200 mg per day. In some embodiments, the dose of magnesium is from about 25 to about 500 mg per day. In some embodiments, the dose of magnesium is equal to about 25, 50, 100, 150, 200, 250, 300, 350, 400, 450 or 500 mg per day or within a range defined by any two of the aforementioned values. The dose of magnesium in the topical composition may be greater than about 1 mg per day and less than about 1 g per day. For example, the topical dose of magnesium may be from about 1 mg to about 1 g per day, e.g. from about 25 mg to about 500 mg per day, such as from about 50 mg to about 450 mg per day, e.g. from about 50 mg to about 400 mg per day, such as from about 100 mg to about 350 mg per day, e.g. from about 100 mg to about 300 mg per day, such as from about 150 mg to about 250 mg per day, e.g. about 200 mg per day.

In another variation, the topical composition comprises magnesium in a range of about 40 mg to about 400 mg per 1/2 teaspoon of a topical gel or a topical cream. The topical composition may comprise from about 40 mg to about 400 mg magnesium per 2.5 mL, such as from about 100 mg to about 300 mg per 2.5 mL, e.g. about 200 mg magnesium per 2.5 mL. The topical composition is preferably a topical gel or topical cream.

In some embodiments, the topical composition is used for about 1 day to about 21 days. In some embodiments, the topical composition is used for 1, 2, 3, 4, 5, 6, 7, 8, 9, 10, 11, 12, 13, 14, 15, 16, 17, 18, 19, 20 or 21 days or within a range defined by any two of the aforementioned values. In some embodiments, the topical composition is used for about 1 to about 52 weeks. In some embodiments, the topical composition is used for 4, 5, 6, 7, 8, 9, 10, 11, 12, 13, 14, 15, 16, 17, 18, 19, 20, 21, 22, 23, 24, 25, 26, 27, 28, 29 or 30 weeks or within a range defined by any two of the aforementioned values. For example, the topical composition may be used for from about 1 day to about 52 weeks, such as from about 2 days to about 30 weeks, such as from about 3 days to about 24 weeks, e.g. from about 4 days to about 20 weeks, such as from about 5 days to about 16 weeks, e.g. from about 6 days to about 12 weeks, such as from about 7 days to about 8 weeks, e.g. from about 8 days to about 7 weeks, for example from about 9 days to about 6 weeks, e.g. from about 10 days to about 5 weeks, such as from about 12 days to about 4 weeks, e.g. from about 14 days to about 3 weeks (21 days), such as from about 15 to about 20 days, e.g. from about 16 or 17 to about 18 or 19 days.

The topical composition may be used as divided doses per day. In some embodiments, the topical composition is used as about 2 to about 12 divided doses per day. In some embodiments, the topical composition is used as 2, 3, 4, 5, 6, 7, 8, 9, 10, 11 or 12 divided doses per day or within a range defined by any two of the aforementioned values. In some embodiments, the topical composition is used as multiple doses per day. In some embodiments, the topical composition is used as about 2 to about 12 multiple doses per day. In some embodiments, the topical composition is used as 2, 3, 4, 5, 6, 7, 8, 9, 10, 11 or 12 multiple doses per day or within a range defined by any two of the aforementioned values. For example, the topical composition may be used as from about 2 to about 12 divided doses per day, such as from about 3 to about 10, e.g. from about 4 to about 8, such as from about 5 to about 6 divided doses per day.

In some embodiments, the age of the subject is from about 10 to about 90 years. The age of the subject may be 10, 15, 20, 25, 30, 35, 40, 45, 50, 55, 60, 65, 70, 75, 80, 85 or 90 years or within a range defined by any two of the aforementioned values. In some embodiments, the age of the subject is lower than about 10 years. In some embodiments, the age of the subject is higher than about 70 years. In some embodiments, the sex of the subject is a male. In some embodiments, the sex of the subject is a female. In some embodiments, the subject has had rosacea for about 1 day. In some embodiments, the subject has had rosacea for less than 5 years. In some embodiments, the subject has had rosacea for about 5 years. In some embodiments, the subject has had rosacea for more than about 5 years. For example, the subject may have had rosacea for about 1 day to about 10 years, such as from about 1 year to about 5 years, e.g. about 2 to about 3 or 4 years. In some embodiments, the weight of the subject is from about 100 to about 250 lbs. In some embodiments, the weight of the subject is lower than about 100 lbs. In some embodiments, the weight of the subject is higher than about 250 lbs.

In some embodiments, the subject has not tried other rosacea remedies. In some embodiments, the subject has tried other rosacea remedies. In some embodiments, the subject has unsuccessfully tried other rosacea remedies. In some embodiments, the relief from rosacea from other remedies was less than about 50%. In some embodiments, the relief from rosacea from other remedies was about 5 to about 50%. In some embodiments, the relief from rosacea from other remedies was equal to about 5, 10, 15, 20, 25, 30, 35, 40, 45 or 50% or within a range defined by any two of the aforementioned values.

In some embodiments, the relief from rosacea from topical TAUMAG-R is about 30 to about 100%. In some embodiments, the relief from rosacea from topical TAUMAG-R may be equal to about 30, 35, 40,45, 50, 55, 60, 65, 70, 75, 80, 85, 90, 91, 92, 93, 94, 95, 96, 97, 98, 99 or 100% or within a range defined by any two of the aforementioned values. For example, the subject may have obtained about 5 to about 50%, e.g. from about 10 to about 40%, such as from about 20 to about 30% relief from rosacea from other remedies, and/or the subject may obtain from about 30 to about 100%, such as from about 40 to about 99%, e.g. from about 50 to about 98%, such as from about 60 to about 97%, e.g. from about 70 to about 95%, such as from about 80 to about 90% relief from the topical composition.

In some embodiments, the topical composition additionally may comprise other micronutrients such as vitamins. In some embodiments, the topical composition additionally may comprise other micronutrients such as minerals. In some embodiments, the topical composition additionally may comprise other vitamins and minerals. In some embodiments, the topical composition additionally may comprise one or more micronutrients, wherein the one or more micronutrients is selected from the group consisting of vitamin A, vitamin B1, vitamin B2, vitamin B3, vitamin B5, vitamin B6, vitamin B12, vitamin C, vitamin D, vitamin E, vitamin K, Folic Acid, Biotin, Calcium, Iron, Phosphorous, Iodine, Potassium, Zinc, Selenium, Manganese, Copper, Choline, Inositol, Omega 3 fatty acids, Lycopene, Lutein and Zeaxanthin.

In some embodiments, the subject has mild rosacea. In some embodiments, the subject has moderate rosacea. In some embodiments, the subject has severe rosacea. In some embodiments, the subject has mild-moderate rosacea. In some embodiments, the subject has moderate-severe rosacea. In some embodiments, topical TAUMAG-R may be partially effective. In some embodiments, topical TAUMAG-R may be about 30 to about 99% effective. In some embodiments, topical TAUMAG-R is 30,40, 50, 60, 70, 80, 85, 90, 91, 92, 93, 94, 95, 96, 97, 98 or 99% effective or within a range defined by any two of the aforementioned values. In some embodiments, topical TAUMAG-R is fully effective. In some embodiments, topical TAUMAG-R may be about 100% effective. Topical TAUMAG-R may mitigate scars due to rosacea. Topical TAUMAG-R may mitigate scars due to mild rosacea. Topical TAUMAG-R may mitigate scars due to moderate rosacea. Topical TAUMAG-R may mitigate scars due to severe rosacea. Scarring from rosacea may be reduced about 20 to about 80% by topical TAUMAG-R. Scarring from rosacea may be reduced equal to about 20, 25, 30, 35, 40, 45, 50, 55, 60, 65, 70, 75 or 80% by topical TAUMAG-R or within a range defined by any two of the aforementioned values. For example, the topical composition may decrease scarring from rosacea by about 20 to about 80%, such as from about 30 to about 70%, e.g. from about 40 to about 60%, e.g. about 50%.

In some embodiments, topical compositions comprising high-dose taurine (about 3 g per day) together with low-dose magnesium (about 300 mg per day) may be very effective against mild-moderate rosacea, and may be partially effective against severe rosacea.

In some embodiments, topical compositions comprising high-dose taurine (about 3 g per day, in divided doses) in combination with low-dose magnesium (about 300 mg magnesium per day, in divided doses) may improve mild-moderate rosacea about 30 to about 100% within about 4 to about 12 weeks.

The formulation may be a topical anti-rosacea gel/cream. The goal of applying a topical formulation is to achieve rapid penetration of taurine and magnesium into the pilosebaceous units of the skin. Depending on the severity of rosacea, either an oral formulation or a topical formulation or both an oral and a topical formulation may be used by the subject in need thereof. In some cases, the oral and/or topical formulation may be combined with other micronutrients such as vitamins and/or minerals.

In some variations, topical TAUMAG-R may be a squeezable tube or a jar, of any pharmacological size and dimension, of anti-rosacea topical gel or cream. In some embodiments, contents of topical TAUMAG-R are taurine, magnesium and a vehicle (pharmaceutical carrier of dermatological agents) gel or cream comprised of inactive ingredients and preferably aqueous-based. For each 1/2 teaspoon (2.5 mL) of topical TAUMAG-R gel/cream, the amount of taurine contained in it may be in a range of about 200 mg to about 2 g, and the amount of magnesium contained in it may be in a range of about 20 mg to about 400 mg. In some embodiments, the topical composition comprises TAUMAG-R dissolved in an anhydrous or nonaqueous solvent. An example is a solvent comprising a monohydric aliphatic alcohol and a polyol.

The composition of the vehicle may be selected so as to be easy to apply and remove, be nonirritant, non-allergenic, chemically stable, homogenous, and pharmacologically inert. In some embodiments, vehicle gels/creams such as this are well known in pharmaceutical and aesthetics industries by those skilled in the art of manufacturing gels/creams. In some embodiments, taurine and magnesium would be mixed into the vehicle to form the gel/cream. As an example, the use of topical TAUMAG-R is contemplated to be as follows: a subject in need of topical TAUMAG-R would collect about 1/8 to about 1/2 teaspoon (0.625-2.5 mL) of gel/cream either from a tube of topical TAUMAG-R or from a jar of topical TAUMAG-R on a suitable device and apply it to an affected area of the body. The affected area can be the face, parts of the neck, chest, and back, and/or other affected bodily areas.

Treatment compositions based on nanoparticles are also contemplated as novel treatments of rosacea and other skin lesions. Because of their unique physical properties, including their high surface area to size ratio, nanoparticles are ideal for use in various skin care products currently on the market because of their ability for enhanced mobility within any environment in the body. The benefits, side-effects and long-term consequences of using nanoparticles in therapeutic and/or cosmetic compositions are currently being actively explored (Wiesenthal A et al., International Journal of Dermatology, Mar;50(3):247-54 (2011)).

Thus, in some embodiments, the composition may be topically delivered using nanoparticles. The nanoparticles comprise those that can be used for dermatological purposes. In some embodiments, nanoparticles can be used for topical spot treatment of, without limitation, one or more of mild/sporadic or break-out lesions, acute impending lesions or severe rosacea.

It is understood in the art that an active ingredient in a composition may require a different vehicle for optimized therapy depending on the concentration of the active ingredient used for therapy. Therefore, one of ordinary skill in the art may decide which vehicle to use on a case by case basis. An example of inactive ingredients in a topical composition may comprise carbomer (gelling agent), purified water (solvent), potassium sorbate (preservative), propylene glycol (permeation enhancer). Additional examples of excipients in topical formulations can be found in Chang et al., The AAPS Journal, 15:41-52 (2013), which is hereby incorporated by reference in its entirety. In some embodiments, the inactive ingredients may be selected from acrylates copolymer, carbomer 940, docusate sodium, edetate disodium, glycerin, poloxamer 182, propylene glycol, purified water, silicon dioxide, sodium hydroxide. In another variation, inactive ingredients in a topical composition may comprise carbomer, disodium EDTA, hydroxypropyl methylcellulose, laureth-4, sodium hydroxide, water. Additional examples of topical formulations can be found in Raphael et al., Therapeutic Delivery, Feb 6, 2:197-216 (2015), which is hereby incorporated by reference in its entirety.

### Oral Compositions Comprising Taurine and Magnesium for Rosacea

In some embodiments an oral composition or combination is provided. In some embodiments, the oral composition or combination comprises taurine and magnesium. The oral composition may be referred to herein as oral TAUMAG-R. In some embodiments, the oral TAUMAG-R is an oral supplement comprising high-dose taurine and low-dose magnesium. In some embodiments, the oral composition comprises a high dose of taurine. The magnesium in TAUMAG-R may be magnesium citrate, magnesium chloride, magnesium oxide or some other form of magnesium. In some embodiments, the dose of taurine is about 3 g per day. In some embodiments, the dose of taurine is from about 0.5 to about 6 g per day. In some embodiments, the dose of taurine is equal to about 0.5, 1, 1.5, 2, 2.5, 3, 3.5, 4, 4.5, 5, 5.5 or 6 g per day or within a range defined by any two of the aforementioned values. The dose of taurine in the oral composition may be greater than about 0.1 g per day and less than about 20 g per day. In some embodiments, the dose of magnesium is about 200 mg per day. In some embodiments, the dose of magnesium is from about 25 to about 500 mg per day. In some embodiments, the dose of magnesium is equal to about 25, 50, 100, 150, 200, 250, 300, 350, 400, 450 or 500 mg per day or within a range defined by any two of the aforementioned values. For example, an oral dose of taurine may be from about 0.1 g to about 20 g per day, e.g. from about 0.5 g to about 6 g per day, such as from about 1 g to about 5.5 g per day, e.g. from about 1.5 g to about 5 g per day, such as from about 2 g to about 4.5 g per day, e.g. from about 2.5 g to about 4 g per day, such as from about 3 g to about 3.5 g per day.

In another variation, the oral supplement is a tablet or a capsule or a powder comprising taurine in a range of about 500 to about 1500 mg per unit dose, e.g. per capsule or per tablet or per unit dose of powder. The oral composition may comprise from about 200 mg to about 2000 mg taurine per unit dose, such as from about 500 mg to about 1500 mg per unit dose, such as about 1000 mg per unit dose. The oral composition is preferably provided as a unit dose consisting of or comprising a capsule or per tablet or per unit dose of powder.

In some embodiments, the oral composition additionally comprises a low dose of magnesium. The dose of magnesium in the oral composition or combination may be greater than about 1 mg per day and less than about 1 g per day. For example, the oral dose of magnesium may be from about 1 mg to about 1 g per day, e.g. from about 25 mg to about 700 mg per day, such as from about 50 mg to about 600 mg per day, e.g. from about 100 mg to about 500 mg per day, such as from about 150 mg to about 450 mg per day, e.g. from about 200 mg to about 400 mg per day, such as from about 250 mg to about 350 mg per day, e.g. about 300 mg per day.

In another variation, the oral supplement is a unit dose such as a tablet or a capsule or a powder comprising magnesium in a range of about 50 to about 150 mg per unit dose, e.g. per capsule or per tablet or per unit dose of powder. The oral composition may comprise magnesium from about 50 mg to about 150 mg per unit dose, such as about 100 mg per unit dose. The oral composition is preferably a capsule or a tablet or a unit dose of powder.

In some embodiments, the oral composition is taken for about 1 day to about 21 days. In some embodiments, the oral composition is taken for 1, 2, 3, 4, 5, 6, 7, 8, 9, 10, 11, 12, 13, 14, 15, 16, 17, 18, 19, 20 or 21 days or within a range defined by any two of the aforementioned values. In some embodiments, the oral composition is taken for about 1 to about 52 weeks. In some embodiments, the oral composition is taken for 4, 5, 6, 7, 8, 9, 10, 11, 12, 13, 14, 15, 16, 17, 18, 19, 20, 21, 22, 23, 24, 25, 26, 27, 28, 29 or 30 weeks or within a range defined by any two of the aforementioned values. For example, the oral composition may be used for from about 1 day to about 52 weeks, such as from about 3 days to about 40 weeks, such as from about 5 days to about 30 weeks, e.g. from about 10 days to about 20 weeks, such as from about 12 days to about 18 weeks, e.g. from about 14 days to about 16 weeks, such as from about 16 days to about 14 weeks, e.g. from about 18 days to about 13 weeks, for example from about 20 days to about 12 weeks.

In some embodiments, the oral composition is taken as divided doses per day. In some embodiments, the oral composition is taken as about 2 to about 12 divided doses per day. In some embodiments, the oral composition is taken as 2, 3, 4, 5, 6, 7, 8, 9, 10, 11 or 12 divided doses per day or within a range defined by any two of the aforementioned values. For example, the oral composition may be used as from about 2 to about 12 divided doses per day, such as from about 3 to about 6, e.g. from about 4 to about 5 divided doses per day.

In some embodiments, the oral composition is taken as multiple doses per day. In some embodiments, the oral composition is taken as about 2 to about 12 multiple doses per day. In some embodiments, the oral composition is taken as 2, 3, 4, 5, 6, 7, 8, 9, 10, 11 or 12 multiple doses per day or within a range defined by any two of the aforementioned values. In some embodiments, the oral combination of taurine and magnesium may comprise separate formulations of each active ingredient. In some embodiments, the oral TAUMAG-R for rosacea is taken on an empty stomach or a near-empty stomach. In other words, when used to treat or prevent rosacea in a subject in need thereof, the oral composition is typically administered to the subject when the subject has an empty or near-empty stomach.

In some embodiments, the age of the subject is from about 10 to about 90 years. In some embodiments, the age of the subject is 10, 15, 20, 25, 30, 35, 40, 45, 50, 55, 60, 65, 70, 75, 80, 85 or 90 years or within a range defined by any two of the aforementioned values. In some embodiments, the age of the subject is lower than about 10 years. In some embodiments, the age of the subject is higher than about 70 years. In some embodiments, the sex of the subject is a male. In some embodiments, the sex of the subject is a female. For example, the subject may be a male or female subject of age from about 10 to about 90 years, such as from about 20 to about 80, e.g. from about 30 to about 70, such as from about 40 to about 60, e.g. about 50 years.

In some embodiments, the subject has had rosacea for about 1 day. In some embodiments, the subject has had rosacea for less than 5 years. In some embodiments, the subject has had rosacea for about 5 years. In some embodiments, the subject has had rosacea for more than about 5 years. For example, the subject may have had rosacea for about 1 day to about 10 years, such as from about 1 year to about 5 years, e.g. about 2 to about 3 or 4 years. In some embodiments, the weight of the subject is from about 100 to about 250 lbs. In some embodiments, the weight of the subject is lower than about 100 lbs. In some embodiments, the weight of the subject is higher than about 250 lbs.

In some embodiments, the subject has not tried other rosacea remedies. In some embodiments, the subject has tried other rosacea remedies. In some embodiments, the subject has unsuccessfully tried other rosacea remedies. In some embodiments, the relief from rosacea from other remedies was less than about 50%. In some embodiments, the relief from rosacea from other remedies was about 5 to about 50%. In some embodiments, the relief from rosacea from other remedies was equal to about 5, 10, 15, 20, 25, 30, 35, 40, 45 or 50% or within a range defined by any two of the aforementioned values.

In some embodiments, the relief from rosacea from oral TAUMAG-R was about 30 to about 100%. In some embodiments, the relief from rosacea from oral TAUMAG-R was equal to about 30, 35, 40,45, 50, 55, 60, 65, 70, 75, 80, 85, 90,91, 92, 93, 94, 95, 96, 97, 98, 99 or 100% or within a range defined by any two of the aforementioned values. For example, the subject may have obtained about 5 to about 50%, e.g. from about 10 to about 40%, such as from about 20 to about 30% relief from rosacea from other remedies, and/or the subject may obtain from about 30 to about 100%, such as from about 40 to about 99%, e.g. from about 50 to about 98%, such as from about 60 to about 97%, e.g. from about 70 to about 95%, such as from about 80 to about 90% relief from the oral composition.

Oral TAUMAG-R may mitigate scars due to rosacea. Oral TAUMAG-R may mitigate scars due to mild rosacea. Oral TAUMAG-R may mitigate scars due to moderate rosacea. Oral TAUMAG-R may mitigate scars due to severe rosacea. Scarring from rosacea may be reduced by about 20% to about 80% by oral TAUMAG-R. Scarring from rosacea may be reduced by about 20, 25, 30, 35, 40, 45, 50, 55, 60, 65, 70, 75 or 80% by oral TAUMAG-R or within a range defined by any two of the aforementioned values. For example, the oral composition may decrease scarring from rosacea by about 20 to about 80%, such as from about 30 to about 70%, e.g. from about 40 to about 60%, e.g. about 50%.

In some embodiments, the oral composition or combination additionally comprises other micronutrients such as vitamins. In some embodiments, the oral composition additionally comprises other micronutrients such as minerals. In some embodiments, the oral composition additionally comprises other vitamins and minerals. In some embodiments, the oral composition additionally comprises one or more micronutrients, wherein the one or more micronutrients is selected from the group consisting of vitamin A, vitamin B1, vitamin B2, vitamin B3, vitamin B5, vitamin B6, vitamin B12, vitamin C, vitamin D, vitamin E, vitamin K, Folic Acid, Biotin, Calcium, Iron, Phosphorous, Iodine, Potassium, Zinc, Selenium, Manganese, Copper, Choline, Inositol, Omega 3 fatty acids, Lycopene, Lutein and Zeaxanthin.

In some embodiments, the subject has mild rosacea. In some embodiments, the subject has moderate rosacea. In some embodiments, the subject has severe rosacea. In some embodiments, the subject has mild-moderate rosacea. In some embodiments, the subject has moderate-severe rosacea. In some embodiments, oral TAUMAG-R is partially effective. In some embodiments, oral TAUMAG-R is about 30 to about 99% effective. In some embodiments, oral TAUMAG-R is 30, 35, 40, 45, 50, 55, 60, 65, 70, 75, 80, 85, 90, 91, 92, 93, 94, 95, 96, 97, 98 or 99% effective or within a range defined by any two of the aforementioned values. In some embodiments, oral TAUMAG-R is fully effective. In some embodiments, oral TAUMAG-R is about 100% effective.

In some embodiments, oral supplements comprising high-dose taurine (about 3 g per day) together with low-dose magnesium (about 300 mg per day) can be very effective against mild-moderate rosacea, and can be partially effective against severe rosacea.

In some embodiments, oral supplementation with high-dose taurine oral supplementation (about 3 g per day, in divided doses) in combination with low-dose magnesium (about 300 mg magnesium per day, in divided doses) improves mild-moderate rosacea about 30 to about 100% within about 4 to about 12 weeks in adults.

In some embodiments, oral TAUMAG-R is a formulation such as a capsule, caplet, tablet, other pill form, or powder. A subject in need of the formulation can take about 0.25--about 12 unit doses per day. The unit dose can be taken as a single dose or can be taken as a divided dose. The active ingredients of the formulation are a high dose of taurine and a low dose of magnesium. In some embodiments, each unit dose of the formulation comprises between about 200 mg to about 2 g taurine and about 50 mg to about 150 mg magnesium. The dose of taurine may be about 3 g per day. In some cases, the dose of taurine may range from about 0.5 to about 6 g per day. In some embodiments, the dose of taurine is equal to about 0.5, 1, 1.5, 2, 2.5, 3, 3.5, 4, 4.5, 5, 5.5 or 6 g per day or within a range defined by any two of the aforementioned values. The dose of taurine in the oral composition may be greater than about 0.1 g per day and less than about 20 g per day. In all embodiments, the oral composition additionally comprises a low dose of magnesium. In some embodiments, the dose of magnesium is about 200 mg per day. In some embodiments, the dose of magnesium is from about 25 to about 500 mg per day. In some embodiments, the dose of magnesium is equal to about 25, 50, 100, 150, 200, 250, 300, 350, 400, 450 or 500 mg per day or within a range defined by any two of the aforementioned values. The dose of magnesium in the oral composition may be greater than about 1 mg per day and less than about 1 g per day. The dose of magnesium may be equal to about 25, 50, 100, 150, 200, 250, 300, 350, 400, 450 or 500 mg per day or within a range defined by any two of the aforementioned values.

In addition to the active ingredients, the formulation may have inactive ingredients, such as a coating of pills, coloring agents for capsules, etc. The coating may comprise standard coating ingredients such as magnesium stearate and cellulose. The coating may be such that it allows for a timed-release of the active ingredients. The formulation may be sold in a pharmaceutical/nutraceutical bottle.

It is understood in the art that an active ingredient in a composition may require a different vehicle for optimized therapy depending on the concentration of the active ingredient used for therapy. Therefore, one of ordinary skill in the art may decide which vehicle to use on a case by case basis. An example of inactive ingredients in an oral composition may comprise cellulose (stabilizer, thickener), magnesium stearate (flow agent), and silicon dioxide (glidant). Additional examples of excipients in oral formulations can be found in Dave, Drug Topics, Oct. 24, 2008, which is hereby incorporated by reference in its entirety. Additional examples of oral formulations can be found in Mitragotri et al., Nature Reviews Drug Discovery, 13:655-672 (2014), which is hereby incorporated by reference in its entirety.

### Additional Embodiments

In some embodiments, an anti-rosacea kit is provided. The anti-rosacea kit may comprise only topical TAUMAG-R. The kit may comprise only oral TAUMAG-R. In another variation, the kit may comprise both topical TAUMAG-R and oral TAUMAG-R. The kit may contain topical TAUMAG-R and an oral supplement comprising various other micronutrients such as vitamins and minerals that may aid in effectiveness of taurine and magnesium against rosacea. The kit may contain oral TAUMAG-R and an oral supplement comprising various other micronutrients such as vitamins and minerals that may aid in effectiveness of taurine and magnesium against rosacea. The kit may comprise topical TAUMAG-R, oral TAUMAG-R and an oral supplement comprising various other micronutrients such as vitamins and minerals that may aid in effectiveness of taurine and magnesium against rosacea. In some embodiments, the kit may comprise enough topical TAUMAG-R and oral TAUMAG-R to provide an average user with about 2-16 weeks of treatment. In some embodiments, the topical and oral TAUMAG-R combinations may comprise separate formulations of topical and oral taurine and separate formulations of topical and oral magnesium.

The main reason to provide a kit of topical TAUMAG-R and oral TAUMAG-R that additionally comprises oral supplements from among an assortment of other micronutrients is to help prevent micronutrient deficiencies that could interfere with treatment of the skin. Most micronutrients are known to have effects on the skin, so trying to ensure that the skin obtains adequate levels of such micronutrients could potentially help with the ability of topical TAUMAG-R and oral TAUMAG-R to heal and/or prevent rosacea. Thus, in some embodiments, a kit is provided that comprises topical TAUMAG-R and oral TAUMAG-R, and additionally comprises oral supplements from among an assortment of other micronutrients, in order to help prevent micronutrient deficiencies that might interfere with treatment of the skin. Most micronutrients are important for some aspect of skin health, such as the healing of lesions. And certain micronutrients appear to support the specific functions of taurine and magnesium in the pilosebaceous units and the stratum corneum. For example, vitamin D, vitamin A and zinc appear to facilitate taurine transport across cell membranes. Since a deficiency in such supportive micronutrients could hinder the ability of taurine and magnesium to adequately perform their functions in the pilosebaceous units and stratum corneum, supplementation with these micronutrients during TAUMAG-R treatment may aid in the reduction and/or prevention of rosacea. The oral supplements of additional micronutrients for the kit could resemble a standard multivitamin/mineral supplement, with doses selected to achieve optimal skin health or to best complement the taurine and the magnesium.

The oral supplement of micronutrients may additionally comprise micronutrients such as vitamins and/or minerals. The micronutrients are selected from the group consisting of vitamin A, vitamin B1, vitamin B2, vitamin B3, vitamin B5, vitamin B6, vitamin B12, vitamin C, vitamin D, vitamin E, vitamin K, Folic Acid, Biotin, Calcium, Iron, Phosphorous, Iodine, Potassium, Zinc, Selenium, Manganese, Copper, Choline, Inositol, Omega 3 fatty acids, Lycopene, Lutein and Zeaxanthin.

In some embodiments, an oral treatment or a topical treatment of an infant or baby who has rosacea is provided. The oral or topical treatment may be used for prevention of rosacea in the infant or baby. The oral or topical treatment has a high dose of taurine and a low dose of magnesium. Typically, unless otherwise specified, the oral treatment is provided as an oral composition as described in more detail herein. Typically, unless otherwise specified, the topical treatment is provided as a topical composition as described in more detail herein. In some embodiments, the age of the infant or baby can range from about 1 day to about 2 years. The infant or baby can be a male. The infant or baby can be a female. In some embodiments, the infant or baby has had rosacea for about 1 day. The infant or baby may have had rosacea for about 2 years. The weight of the infant or baby may range from about 2 to about 30 lbs. The weight of the infant or baby may be lower than about 2 lbs. The weight of the infant or baby may be higher than about 30 lbs.

In the oral or topical treatment for an infant or baby, the dose of taurine is about 0.15 g per day. In some cases, the dose of taurine may range from about 0.05 to about 0.6 g per day. In some embodiments, the dose of taurine is equal to about 0.05, 0.1, 0.15, 0.2, 0.25, 0.3, 0.35, 0.4, 0.45, 0.5, 0.55 or 0.6 g per day or within a range defined by any two of the aforementioned values (e.g. from about 0.05 to about 0.6 g per day, such as from about 0.1 to about 0.4 g per day, e.g. from about 0.15 to about 0.25 g per day). The dose of taurine in the oral composition may be greater than about 0.01 g per day and less than about 2 g per day. In all embodiments, the oral composition additionally comprises a low dose of magnesium. In some embodiments, the dose of magnesium is about 50 mg per day. In some embodiments, the dose of magnesium is from about 6.25 to about 125 mg per day. In some embodiments, the dose of magnesium is equal to about 6.25, 12.5, 25, 37.5, 50, 62.5, 75, 87.5, 100, 112.5 or 125 mg per day or within a range defined by any two of the aforementioned values (e.g. from about 6.25 to about 125 mg per day, such as from about 25 to about 75 mg per day, e.g. from about 50 to about 62.5 mg per day). The dose of magnesium in the oral composition may be greater than about 0.25 mg per day and less than about 0.25 g per day. The oral composition for the infant or baby may be an oral supplement, for example a special baby formula with TAUMAG-R for oral consumption specifically for baby skin conditions. In some embodiments, the oral composition is taken for about 1 day to about 21 days. In some embodiments, the oral composition is taken for 1, 2, 3, 4, 5, 6, 7, 8, 9, 10, 11, 12, 13, 14, 15, 16, 17, 18, 19, 20 or 21 days or within a range defined by any two of the aforementioned values. In some embodiments, the oral composition is taken for about 1 to about 52 weeks. In some embodiments, the oral composition is taken for 4, 5, 6, 7, 8, 9, 10, 11, 12, 13, 14, 15, 16, 17, 18, 19, 20, 21, 22, 23, 24, 25, 26, 27, 28, 29 or 30 weeks or within a range defined by any two of the aforementioned values. For example, the composition can be used for from about 1 day to about 52 weeks, such as from about 7 days to about 8 weeks, e.g. from about 14 days to about 3 weeks.

In some embodiments, an oral treatment or a topical treatment of a young child or pre-teen is provided. The oral or topical treatment may be used for prevention of rosacea in the young child or pre-teen. The oral treatment has a high dose of taurine and a low dose of magnesium. Typically, unless otherwise specified, the oral treatment is provided as an oral composition as described in more detail herein. Typically, unless otherwise specified, the topical treatment is provided as a topical composition as described in more detail herein. In some embodiments, the age of the young child or pre-teen can range from about 2 years to about 12 years. The young child or pre-teen can be a male. The young child or pre-teen can be a female. The young child or pre-teen may have had rosacea for about 1 day. The young child or pre-teen may have had rosacea for about 2 years. The weight of the young child or pre-teen may range from about 30 to about 120 lbs. The weight of the young child or pre-teen may be lower than about 30 lbs. The weight of the young child or pre-teen may be higher than about 120 lbs.

In the oral or topical treatment for a young child or pre-teen, the dose of taurine is about 1.5 g per day. In some cases, the dose of taurine may range from about 0.5 to about 6 g per day. In some embodiments, the dose of taurine is equal to about 0.5, 1, 1.5, 2, 2.5, 3, 3.5, 4, 4.5, 5, 5.5 or 6 g per day or within a range defined by any two of the aforementioned values (e.g. from about 0.5 to about 6 g per day, such as from about 1 to about 4 g per day, e.g. from about 1.5 to about 2.5 g per day). The dose of taurine in the oral composition may be greater than about 0.1 g per day and less than about 20 g per day. In some embodiments, the oral composition additionally comprises a low dose of magnesium. In some embodiments, the dose of magnesium is about 200 mg per day. In some embodiments, the dose of magnesium is from about 25 to about 500 mg per day. In some embodiments, the dose of magnesium is equal to about 25, 50, 100, 150, 200, 250, 300, 350, 400, 450 or 500 mg per day or within a range defined by any two of the aforementioned values (e.g. from about 25 to about 500 mg per day, such as from about 100 to about 300 mg per day, e.g. from about 200 to about 250 mg per day). The dose of magnesium in the oral composition may be greater than about 1 mg per day and less than about 1 g per day. In some embodiments, the oral composition is taken for about 1 day to about 21 days. In some embodiments, the oral composition is taken for 1, 2, 3, 4, 5, 6, 7, 8, 9, 10, 11, 12, 13, 14, 15, 16, 17, 18, 19, 20 or 21 days or within a range defined by any two of the aforementioned values. In some embodiments, the oral composition is taken for about 1 to about 52 weeks. In some embodiments, the oral composition is taken for 4, 5, 6, 7, 8, 9, 10, 11, 12, 13, 14, 15, 16, 17, 18, 19, 20, 21, 22, 23, 24, 25, 26, 27, 28, 29 or 30 weeks or within a range defined by any two of the aforementioned values. For example, the composition can be used for from about 1 day to about 52 weeks, such as from about 7 days to about 8 weeks, e.g. from about 14 days to about 3 weeks.

In some embodiments, an oral treatment or a topical treatment of a teen is provided. The oral or topical treatment may be used for prevention of rosacea in the teen. The oral treatment has a high dose of taurine and a low dose of magnesium. Typically, unless otherwise specified, the oral treatment is provided as an oral composition as described in more detail herein. Typically, unless otherwise specified, the topical treatment is provided as a topical composition as described in more detail herein. In some embodiments, the age of the teen can range from about 13 years to about 19 years. The teen can be a male. The teen can be a female. The teen may have had rosacea for about 1 day. The teen may have had rosacea for about 2 years. The weight of the teen may range from about 80 to about 280 lbs. The weight of the teen may be lower than about 80 lbs. The weight of the teen may be higher than about 280 lbs.

In the oral or topical treatment for a teen, the dose of taurine is about 1.5 g per day. In some cases, the dose of taurine may range from about 0.5 to about 6 g per day. In some embodiments, the dose of taurine is equal to about 0.5, 1, 1.5, 2, 2.5, 3, 3.5, 4, 4.5, 5, 5.5 or 6 g per day or within a range defined by any two of the aforementioned values (e.g. from about 0.5 to about 6 g per day, such as from about 1 to about 4 g per day, e.g. from about 1.5 to about 2.5 g per day). The dose of taurine in the oral composition may be greater than about 0.1 g per day and less than about 20 g per day. In some embodiments, the oral composition additionally comprises a low dose of magnesium. In some embodiments, the dose of magnesium is about 200 mg per day. In some embodiments, the dose of magnesium is from about 25 to about 500 mg per day. In some embodiments, the dose of magnesium is equal to about 25, 50, 100, 150, 200, 250, 300, 350, 400, 450 or 500 mg per day or within a range defined by any two of the aforementioned values (e.g. from about 25 to about 500 mg per day, such as from about 100 to about 450 mg per day, e.g. from about 200 to about 400 mg per day). The dose of magnesium in the oral composition may be greater than about 1 mg per day and less than about 1 g per day. In some embodiments, the oral composition is taken for about 1 day to about 21 days. In some embodiments, the oral composition is taken for 1, 2, 3, 4, 5, 6, 7, 8, 9, 10, 11, 12, 13, 14, 15, 16, 17, 18, 19, 20 or 21 days or within a range defined by any two of the aforementioned values. In some embodiments, the oral composition is taken for about 1 to about 52 weeks. In some embodiments, the oral composition is taken for 4, 5, 6, 7, 8, 9, 10, 11, 12, 13, 14, 15, 16, 17, 18, 19, 20, 21, 22, 23, 24, 25, 26, 27, 28, 29 or 30 weeks or within a range defined by any two of the aforementioned values. For example, the composition can be used for from about 1 day to about 52 weeks, such as from about 7 days to about 20 weeks, e.g. from about 14 days to about 12 weeks.

In some embodiments, any of the oral and/or topical compositions provided herein may comprise one or more of analgesic, anti-pyretic or antihistaminic agents.

In some embodiments, any of the oral and/or topical compositions provided herein may be used as a cosmetic composition for treating one or more dysfunctional pilosebaceous units or the stratum corneum.

In some embodiments, any of the oral and/or topical compositions provided herein may be used as a cosmetic composition in a cosmetic method for treating one or more dysfunctional pilosebaceous units or the stratum corneum.

In some embodiments, any of the topical compositions provided herein may be formulated as a nanoparticle-based cosmetic composition for cosmetically treating one or more dysfunctional pilosebaceous units or the stratum corneum. The one or more nanoparticles efficiently deliver the effective amounts of taurine and magnesium, sufficient in combination, to the one or more dysfunctional pilosebaceous units or the stratum corneum.

In some embodiments, any of the topical compositions provided herein may be used as a nanoparticle-based cosmetic composition in a cosmetic method for cosmetically treating one or more dysfunctional pilosebaceous units or the stratum corneum. The one or more nanoparticles efficiently deliver the effective amounts of taurine and magnesium, sufficient in combination, to the one or more dysfunctional pilosebaceous units or the stratum corneum.

In some embodiments, the nanoparticle can be of zinc oxide, titanium dioxide, gold, silver, platinum, etc. In some embodiments, the nanoparticles can be shaped like nanoplates, rods, shells, wires, prisms, spheres, ovoids, pyramids, cylinders, spirals, cubes, cubiods, ellipsoids, etc. In some embodiments, the nanoparticles can be amorphous.

In some embodiments, the efficiency of a nanoparticle-based composition for cosmetically treating one or more dysfunctional pilosebaceous units or the stratum corneum may be about 50% to about 100% better than of a composition that is not nanoparticle-based. In some embodiments, the efficiency a nanoparticle-based composition for cosmetically treating one or more dysfunctional pilosebaceous units may be about 25, 30, 35, 40, 45, 50, 55, 60, 65, 70, 75, 80, 85, 90, 95, 100, 105, 110, 115, 120, 125, 130, 135, 140, 145 or 150% better than of a composition that is not nanoparticle-based or within a range defined by any two of the aforementioned values.

The standard suggested daily dose of topical TAUMAG-R or oral TAUMAG-R is a safe dose, consisting of an amount of taurine that does not exceed the upper observed safe level that has been reported in the scientific literature in adults, and an amount of magnesium that does not greatly exceed the recommended daily allowance.

### FURTHER EMBODIMENTS

Embodiments may also include the following, as detailed in U.S. Application No. 16/411,025:
1. A combination for treating and/or preventing a skin condition associated with a sebaceous gland disorder, the combination comprising an effective amount of taurine and an effective amount of magnesium, wherein the combination of the effective amounts of taurine and magnesium is sufficient to at least partially normalize sebaceous gland function, and thereby treat and/or prevent the skin condition, and wherein the effective amount of taurine is in a range of about 500 mg to about 6000 mg per unit dose, and the effective amount of magnesium is in a range of about 25 mg to about 500 mg per unit dose.
2. The combination of embodiment 1, wherein the skin condition is acne and/or rosacea.
3. The combination of embodiment 2, wherein the skin condition is rosacea.
4. The combination of embodiment 1, wherein the effective amounts of taurine and magnesium are in a dosage form is selected from an oral or topical dosage form.
5. The combination of embodiment 4, wherein the oral dosage form is selected from a capsule, a tablet or a unit dose of powder.
6. The combination of embodiment 4, wherein the topical dosage form is selected from a gel or a cream.
7. The combination of embodiment 5, wherein the effective amounts of taurine and magnesium are provided together in a single oral dosage form.
8. The combination of embodiment 6, wherein the effective amounts of taurine and magnesium are provided together in a single topical dosage form.
9. The combination of embodiment 1, further comprising a pharmaceutically acceptable carrier.
10. The combination of embodiment 1, further comprising one or more micronutrients selected from the group consisting of vitamin D, vitamin A, zinc, choline, vitamin B1, vitamin B2, vitamin B3, vitamin B5, vitamin C, vitamin E, vitamin K, folic acid, calcium, iron, phosphorous, iodine, potassium, selenium, manganese, copper, inositol, omega 3 fatty acids, lycopene, lutein, and zeaxanthin.
11. A method for treating and/or preventing a skin condition associated with a sebaceous gland disorder in a subject in need thereof, the method comprising administering to the subject an effective amount of taurine and an effective amount of magnesium, simultaneously or sequentially, wherein the effective amounts are sufficient in combination to at least partially normalize sebaceous gland function and/or stratum corneum function, and thereby treat and/or prevent the skin condition, and wherein the effective amount of taurine is in a range of about 500 mg to about 6000 mg per unit dose, and the effective amount of magnesium is in a range of about 25 mg to about 500 mg per unit dose.
12. The method of embodiment 11, wherein the effective amounts of taurine and magnesium are provided in an oral dosage form selected from a capsule, a tablet or a unit dose of powder, formulated for oral administration.
13. The method of embodiment 11, wherein the effective amounts of taurine and magnesium are provided in a topical dosage form selected from a gel or a cream, formulated for topical administration.
14. The method of embodiment 12, wherein the oral dosage form is configured for a single daily dose or multiple daily doses.
15. The method of embodiment 13, wherein the topical dosage form is configured for a single daily dose or multiple daily doses.
16. The method of embodiment 11, further comprising administering one or more micronutrients selected from the group consisting of vitamin D, vitamin A, zinc, choline, vitamin B1, vitamin B2, vitamin B3, vitamin B5, vitamin C, vitamin E, vitamin K, folic acid, calcium, iron, phosphorous, iodine, potassium, selenium, manganese, copper, inositol, omega 3 fatty acids, lycopene, lutein, and zeaxanthin.
17. A combination for treating and/or preventing rosacea, the combination comprising an effective amount of taurine and an effective amount of magnesium, wherein the combination of the effective amounts of taurine and magnesium is sufficient to at least partially normalize stratum corneum function, and thereby treat and/or prevent the rosacea, and wherein the effective amount of taurine is in a range of about 500 mg to about 6000 mg per unit dose, and the effective amount of magnesium is in a range of about 25 mg to about 500 mg per unit dose.
18. A method for treating and/or preventing rosacea in a subject, the method comprising: orally administering to the subject an effective amount of taurine and an effective amount of magnesium simultaneously or sequentially, wherein the effective amounts are sufficient in combination to treat and/or prevent rosacea; and/or topically administering to the subject an effective amount of taurine and an effective amount of magnesium, wherein the effective amounts are sufficient in combination to treat and/or prevent rosacea, wherein the effective amount of taurine is in a range of about 500 mg to about 6000 mg per unit dose, and the effective amount of magnesium is in a range of about 25 mg to about 500 mg per unit dose.

### EXAMPLES

The following Examples illustrate the invention. They do not, however, limit the invention in any way. In this regard, it is important to understand that the effects used in the Examples section are designed only to provide an indication of the efficacy of the invention. There are many assays available to determine effectiveness in treating rosacea, and a negative result in any one particular assay is therefore not determinative.

The following non-limiting examples show Study Volunteer experiences and 'prophetic examples' with various TAUMAG-R treatment regimens.

### Example 1 - Telangiectasia of the Nose

### Subject: Male, age 61

The subject had for approximately 2 years the appearance of very mild rosacea, a chronic slight reddening of the nose. Then very suddenly he developed dark purple telangiectasia of the bulb of the nose that was so pronounced that it was markedly noticeable from 6 feet away. The blood vessels were longer, darker, and thicker than typical in mild rosacea/telangiectasia. There did not seem to be any new factors in his life to account for this sudden facial event. Oral TAUMAG-R was recommended to him, at a starting dose of 2000 mg taurine+200 mg magnesium per day, in 2 daily doses of 1000 mg taurine+100 mg magnesium on an empty stomach. The subject was seen 5 days later. Although he had complied for only 1-2 days with the recommended dose, the dark purple telangiectasia had disappeared. For the next 4 weeks he took oral TAUMAG-R on average one dose per day of 1000 mg taurine+100 mg magnesium and remained clear of dark purple telangiectasia. Close inspection of his face revealed a few faint areas of very thin red or lavender broken blood vessels on the bulb and side of his nose, but no telangiectasia visible from several feet away. After 1 more month of the TAUMAG-R at an average daily dose of about 1200 mg taurine+120 mg magnesium, the reddening of the subject's nasal region from rosacea had disappeared and the texture of the skin there appeared smoother.

The subject continued treatment intermittently for 1 year with excellent results. For several weeks in a row he would take daily either 1 dose of 1000 mg taurine + 100 mg magnesium, or 2 doses totalling 2000 mg taurine + 200 mg magnesium, with the result that the rosacea would disappear almost completely, with only a hint of pink barely noticeable, and no noticeable telangiectasia. Then he would stop treatment for several weeks in a row, with the result that his nose would redden slightly, although without noticeable telangiectasia. Then he would resume treatment again to keep the rosacea at bay.

### Example 2 - ronically Red Facial Skin

### Subject: Female, 56

The subject for more than 3 decades had facial skin that was increasingly red. There was no known underlying systemic medical reason for her marked redness. She suspected rosacea. Oral TAUMAG-R was recommended to her, at a starting dose daily of between 1000 mg + 100 mg magnesium and 2000 mg taurine + 200 mg magnesium. On most days she took only a single dose of 1000 mg taurine + 100 mg magnesium. After 2 months of treatment, her facial redness had disappeared except for a faint pink hue, and her skin had a normal even appearance. Her description was that TAUMAG-R had calmed her facial skin. She stopped treatment to see what would happen. Two months later there was no visible increase in redness. If the redness returns she plans to resume treatment.

### Example 3 -Papulopustular Rosacea (Prophetic Example)

### Subject: Female, age 40

The subject presents with papulopustular rosacea, of degree mild to moderate, such that various regions of the face have red thickened inflamed skin with acne-like eruptions. The subject is administered a daily oral dosage of 3000 mg taurine+300 mg magnesium, in 3 divided doses for eight weeks, followed by a daily maintenance dose of 2000 mg taurine+200 mg magnesium in 2 divided doses. After twelve weeks of daily oral treatment, subject exhibits a significant decrease in sebum volume. In addition, sebum lipid composition is nearly normalized, and partly in consequence, the epidermal lipid composition is nearly normalized. The decrease in sebum volume and normalization of sebum lipid composition is accompanied by clearing and/or shrinking of the papules and pustules, and also decreased infiltration of mites and bacteria that feed on sebum and epidermal lipids. With continued daily maintenance, such treatment is also found to hydrate the cells of the epidermis, decreasing erythema and improving tight junction regulation and barrier protection of the stratum corneum.

This oral TAUMAG-R treatment may also prevent the papulopustular rosacea from progressing to more serious subvariants of rosacea associated with sebaceous problems, such as rhinophyma resulting from sebaceous hyperplasia of the nasal region, and ocular rosacea resulting from the clogging and consequent obstruction of the Meibomian gland, a type of sebaceous gland unique to the eyelids to lubricate the eyes.

### Example 4 - Sebaceous Hyperplasia of Rosacea (Prophetic Example)

### Subject: Male, age 65

The subject presents with sebaceous hyperplasia, involving few or many sebaceous glands or lobules of sebaceous glands. The subject is administered a daily oral dosage of 1000 mg taurine+100 mg magnesium, taken on an empty stomach, as well as a daily topical dosage (in an aqueous gel) of 2000 mg taurine+200 mg magnesium, in 2 divided doses for eight weeks, followed by a topical daily maintenance dose of 1000 mg taurine+100 mg magnesium, in a single dose. The topical gel vehicle includes carbomer, purified water, potassium sorbate, and propylene glycol. Additional micronutrients are included in an oral supplement. After the initial eight weeks, sebaceous gland activity and sebocyte proliferation are significantly decreased, and the size of the sebaceous glands are reduced back toward normal. After one month of daily maintenance dose, inflammation is shown to be reduced. With continued daily maintenance, the topical treatment is also found to hydrate the cells of the epidermis, decreasing erythema and improving tight junction regulation and barrier protection of the stratum corneum.

This topical TAUMAG-R treatment may also prevent the sebaceous hyperplasia associated with rosacea from progressing to the more serious sebaceous problems, such as rhinophyma resulting from severe sebaceous hyperplasia of the nasal region, and ocular rosacea resulting from the clogging and consequent obstruction of the Meibomian gland.

### Definitions

As used herein, the section headings are for organizational purposes only and are not to be construed as limiting the described subject matter in any way. All literature and similar materials cited in this application, including but not limited to, patents, patent applications, articles, books, treatises, and internet web pages are expressly incorporated by reference in their entirety for any purpose. When definitions of terms in incorporated references appear to differ from the definitions provided in the present teachings, the definition provided in the present teachings shall control. It will be appreciated that there is an implied "about" prior to the temperatures, concentrations, times, etc discussed in the present teachings, such that slight and insubstantial deviations are within the scope of the present teachings herein.

In at least some of the previously described embodiments, one or more elements used in an embodiment can interchangeably be used in another embodiment unless such a replacement is not technically feasible. It will be appreciated by those skilled in the art that various other omissions, additions and modifications may be made to the methods and structures described above without departing from the scope of the claimed subject matter. All such modifications and changes are intended to fall within the scope of the subject matter, as defined by the appended claims.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

In this application, the use of the singular includes the plural unless specifically stated otherwise. Also, the use of "comprise", "comprises", "comprising", "contain", "contains", "containing", "include", "includes", and "including" are not intended to be limiting. It is to be understood that both the foregoing general description and the following detailed description are exemplary and explanatory only and are not restrictive. Unless defined otherwise, technical and scientific terms used herein have the same meaning as commonly understood by one of ordinary skill in the art to which this invention belongs.

It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one of skill in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

Although this invention has been disclosed in the context of certain embodiments and examples, those skilled in the art will understand that the present invention extends beyond the specifically disclosed embodiments to other alternative embodiments and/or uses of the invention and obvious modifications and equivalents thereof. In addition, while several variations of the invention have been shown and described in detail, other modifications, which are within the scope of this invention, will be readily apparent to those of skill in the art based upon this disclosure. It is also contemplated that various combinations or sub-combinations of the specific features and aspects of the embodiments may be made and still fall within the scope of the invention. It should be understood that various features and aspects of the disclosed embodiments can be combined with, or substituted for, one another in order to form varying modes or embodiments of the disclosed invention. Thus, it is intended that the scope of the present invention herein disclosed should not be limited by the particular disclosed embodiments described above.

The terminology used in the description presented herein is not intended to be interpreted in any limited or restrictive manner. Rather, the terminology is simply being utilized in conjunction with a detailed description of embodiments of the systems, methods and related components. Furthermore, embodiments may comprise several novel features, no single one of which is solely responsible for its desirable attributes or is believed to be essential to practicing the inventions herein described.

### REFERENCES

All references cited in this disclosure are incorporated herein by reference in their entireties.
Addor FASA. 2016. Skin Barrier in Rosacea. An Bras Dermatol. 91:59-63.
Bickel P E, Tansey J T, Welte M A. 2009. PAT Proteins, an Ancient Family of Lipid Droplet Proteins that Regulate Cellular Lipid Stores. Biochimica et Biophysica Acta. 1791:419-440.
Chandrasekaran N C. 2016. Effect of Topical Magnesium Application on Epidermal Integrity and Barrier Function. PhD Thesis, School of Chemistry and Molecular Biosciences, The University of Queensland, Australia.
Chandrasekaran N C, Sanchez W Y, Mohammed Y H, Grice J E, Roberts M S, Barnard R T. 2016. Permeation of Topically Applied Magnesium Ions Through Human Skin is Facilitated by Hair Follicles. Magnesium Res. 29:35-42.
Chandrasekaran N C, Weir C, Alfraji S, Grice J, Roberts M S, Barnard R T. 2014. Effects of Magnesium Deficiency--More than Skin Deep. Exp Biol Med. 239:1280-1291.
Da Silva D L P, Thiago S B, Pessoa F A, Mrestani Y, Ruettinger H H, Wohlrab J. 2008. Penetration Profile of Taurine in the Human Skin and Its Distribution in Skin Layers. Pharmaceutical Research. 25:1846-1850.
Dahlhoff M, Camera E, Picardo M, Zouboulis C C, Chan L, Chang B H-J, Schneider M R. 2013. PLIN2, the Major Perilipin Regulated During Sebocyte Differentiation, Controls Sebaceous Lipid Accumulation In Vitro and Sebaceous Gland Size In Vivo. Biochimica et Biophysica Acta. 1830:4642-4649.
El-Chami C, Haslam I S, Steward M C, O'Neill C A. 2018. Organic Osmolytes Preserve the Function of the Developing Tight Junction in Ultraviolet B-Irradiated Rat Epidermal Keratinocytes. Sci Rep. 8:5167.12 pages.
Friesen J A, Rodwell VW. 2004. The 3-hydroxy-3-methylglutaryl coenzyme-A (HMG-CoA) reductases. Genome Biology. 5:248
Gomez-Galvez P, Vicente-Munuera P, Tagua A, Forja C, Castro A M, Letran M,Valencia-Exposito A, Grima C, Berm dez-Gallardo M, Serrano-Perez-Higueras O, Cavodeassi F, Sotillos S, Martin-Bermudo M D, Marquez A, Buceta J, Escudero L M. 2018. Scutoids are a Geometrical Solution to Three-Dimensional Packing of Epithelia. Nature Communications. 9:2960.
Hardie D G, Pan D A. 2002. Regulation of Fatty Acid Synthesis and Oxidation by the AMP-Activated Protein Kinase. Biochemical Society Transactions. 30:1064-1070.
Kaushik S, Cuervo A M. 2016. AMPK-Dependent Phosphorylation of Lipid Droplet Protein PLIN2 Triggers its Degradation by CMA. Autophagy. 12:432-438.
Ludovici M, Kozul N, Materazzi S, Risoluti R, Picardo M, Camera E. 2018. Influence of the Sebaceous Gland Density on the Stratum Corneum Lipidome. 2018. Scientific Reports. 8:1-12.
Mughal A, Cox S J, Weaire D, Burke S R, Hutzler S. 2018. Demonstration and Interpretation of "Scutoid" Cells in a Quasi-2D Soap Froth. Philosophical Magazine Letters. 98:1-7.
Mueller J, Oliveira J S L, Barker R, Trapp M, Schroeter A, Brezesinski G, Neubert R H H. 2016 The Effect of Urea and Taurine as Hydrophilic Penetration Enhancers on Stratum Corneum Lipid Models. Biochimica et Biophysica Acta 1858: 2006-2018.
Ni Raghallaigh, Bender K, Lacey N, Brennan L, Powell F C. 2012. The Fatty Acid Profile of the Skin Surface Lipid Layer in Papulopustular Rosacea. British Journal of Dermatology. 166:279-287.
Pappas A. 2009. Epidermal Surface Lipids. Dermato-Endocrinology. 1:72-76.
Rosanoff A, Seelig M S. 2004. Comparison of Mechanism and Functional Effects of Magnesium and Statin Pharmaceuticals. J Am Coll. Nutr. 23:501S-505S.
Sahu-Osen A, Montero-Moran G, Schittmayer M, Fritz K, Dinh A, Chang Y-F, McMahon D, Boeszoermenyi A, Cornaciu I, Russesl D, Oberer M, Carman G M, Birner-Gruenberger R, Brasaemle D L. 2015. CGI-58/ABHDS is Phosphorylated on Ser239 by Protein Kinase A: Control of Subcellular Localization. J Lipid Res. 56:109-121.
Sztalryd C, Xu G, Dorward H, Tansey J T, Contreras J A, Kimmel A R, Londos C. 2003. Perilipin A is Essential for the Translocation of Hormone-Sensitive Lipase during Lipolytic Activation. J Cell Biol. 161:1093-1103.
Tascini A S, Noro M G, Seddon J M, Chen R, Bresme F. 2019. Mechanisms of Lipid Extraction from Skin Lipid Bilayers by Sebum Triglycerides. Phys Chem Chem Phys. 21:1471-1477.
Vemuri R C, Gundamaraju R, Sekaran S D, Manikam R. 2015. Major Pathophysiological Correlations of Rosacea: A Complete Clinical Appraisal. Int j Med Sci. 12:387-396.
Zhou M, Xie H, Cheng L, Li J. 2016. Clinical Characteristics and Epidermal Barrier Function of Papulopustular Rosacea: A Comparison Study with Acne vulgaris. Pak j Med Sci. 32:1344-1348.

## Claims

1. A combination for use in treating and/or preventing a skin condition associated with a sebaceous gland disorder, whereby the skin condition is rosacea, the combination comprising an effective amount of taurine and an effective amount of magnesium, wherein the combination of the effective amounts of taurine and magnesium is sufficient to at least partially normalize sebaceous gland function, and thereby treat and/or prevent the rosacea, and wherein the effective amount of taurine is in a range of about 500 mg to about 6000 mg per unit dose, and the effective amount of magnesium is in a range of about 25 mg to about 500 mg per unit dose.

2. A combination for treating and/or preventing a skin condition associated with a stratum corneum disorder, whereby the skin condition is rosacea, the combination comprising an effective amount of taurine and an effective amount of magnesium, wherein the combination of the effective amounts of taurine and magnesium is sufficient to at least partially normalize stratum corneum function, and thereby treat and/or prevent the rosacea, and wherein the effective amount of taurine is in a range of about 500 mg to about 6000 mg per unit dose, and the effective amount of magnesium is in a range of about 25 mg to about 500 mg per unit dose.

3. The combination for use according to claim 1 and/or 2, wherein the effective amounts of taurine and magnesium are configured for a dosage form that is oral or topical.

4. The combination for use according to claim 3, wherein the oral dosage form is selected from a capsule, a tablet or a unit dose of powder.

5. The combination for use according to claim 3, wherein the topical dosage form is selected from a gel or a cream.

6. The combination for use according to claim 4, wherein the effective amounts of taurine and magnesium are provided together in a single oral dosage form.

7. The combination for use according to claim 5, wherein the effective amounts of taurine and magnesium are provided together in a single topical dosage form.

8. A combination for use in treating and/or preventing rosacea, the combination comprising an effective amount of taurine and an effective amount of magnesium, wherein the dosage form is topical, and wherein the per unit dose of taurine is in a range of 200 mg to 2 g per ½ teaspoon, and the per unit dose of magnesium is in a range of 25 mg to 400 mg per ½ teaspoon.

9. The combination for use according to claim 1 and/or 2 and/or 8, further comprising a pharmaceutically acceptable carrier.

10. The combination for use according to claim 1 and/or 2 and/or 8, further comprising one or more micronutrients selected from the group consisting of vitamin D, vitamin A, zinc, choline, vitamin B1, vitamin B2, vitamin B3, vitamin B5, vitamin C, vitamin E, vitamin K, folic acid, calcium, iron, phosphorous, iodine, potassium, selenium, manganese, copper, inositol, omega 3 fatty acids, lycopene, lutein, and zeaxanthin.

11. A combination for use according to claim 1 and/or 2, wherein said use comprises administering to the subject an effective amount of taurine and an effective amount of magnesium, simultaneously or sequentially, wherein the effective amounts are sufficient in combination to at least partially normalize sebaceous gland function and/or stratum corneum function, and thereby treat and/or prevent the rosacea, and wherein the effective amount of taurine is in a range of about 500 mg to about 6000 mg per unit dose, and the effective amount of magnesium is in a range of about 25 mg to about 500 mg per unit dose.

12. The combination for use according to claim 11, wherein the effective amounts of taurine and magnesium are provided in an oral dosage form selected from a capsule, a tablet or a unit dose of powder, formulated for oral administration.

13. The combination for use according to claim 11, wherein the effective amounts of taurine and magnesium are provided in a topical dosage form selected from a gel or a cream, formulated for topical administration.

14. The combination for use according to claim 12, wherein the oral dosage form is configured for a single daily dose or multiple daily doses.

15. The combination for use according to claim 13, wherein the topical dosage form is configured for a single daily dose or multiple daily doses.

16. The combination for use according to claim 11, further comprising administering one or more micronutrients selected from the group consisting of vitamin D, vitamin A, zinc, choline, vitamin B1, vitamin B2, vitamin B3, vitamin B5, vitamin C, vitamin E, vitamin K, folic acid, calcium, iron, phosphorous, iodine, potassium, selenium, manganese, copper, inositol, omega 3 fatty acids, lycopene, lutein, and zeaxanthin.

17. A combination for use in treating and/or preventing rosacea, wherein said use comprises both topically applying to the subject a dose of the combination in the form of a topical gel or a topical cream, and orally administering to the subject a dose of the combination in the form of an oral supplement.

18. The combination for use according to claim 17, wherein said use comprises: orally administering to the subject an effective amount of taurine and an effective amount of magnesium simultaneously or sequentially, wherein the effective amounts are sufficient in combination to treat and/or prevent rosacea; and topically administering to the subject an effective amount of taurine and an effective amount of magnesium; wherein the effective amounts are sufficient in combination to treat and/or prevent rosacea, wherein the effective amount of taurine is in a range of about 500 mg to about 6000 mg per unit dose, and the effective amount of magnesium is in a range of about 25 mg to about 500 mg per unit dose.

19. The combination for use according to claim 17, wherein said use comprises: orally administering to the subject an effective amount of taurine and an effective amount of magnesium simultaneously or sequentially, wherein the effective amounts are sufficient in combination to at least partially treat and/or prevent rosacea; and topically administering to the subject an effective amount of taurine and an effective amount of magnesium, wherein the effective amounts are sufficient in combination to at least partially treat and/or prevent rosacea, and wherein the per unit dose of topical taurine is in a range of about 200 mg to 2 g per ½ teaspoon, and the per unit dose of topical magnesium is in a range of 25 mg to 400 mg per ½ teaspoon.
